# EUROPEAN PATENT SPECIFICATION

(11) **EP 0 668 877 B1**
(45) Date of publication and mention of the grant of the patent: **20.01.1999**
(21) Application number: 94926756.1
(22) Date of filing: 20.09.1994
(51) Int. Cl.: C07K 14/805, C07K 1/18, A61K 38/42

(54) **DISPLACEMENT CHROMATOGRAPHY PROCESS AND PURIFIED HEMOGLOBIN PRODUCT**
VERDRÄNGUNGSCHROMATOGRAPHISCHES VERFAHREN UND GEREINIGTES HÄMOGLOBIN
PROCEDE DE CHROMATOGRAPHIE PAR DEPLACEMENT ET PRODUIT A BASE D'HEMOGLOBINE PURIFIEE

(30) Priority: 21.09.1993 CA 2106612
(43) Date of publication of application: 30.08.1995
(73) Proprietor: HEMOSOL INC., Etobicoke, Ontario M9W 4Z4 (CA)
(72) Inventor: PLIURA, Diana, Mississauga, Ontario L5M 4M7 (CA); WIFFEN, Diane, Georgetown, Ontario L7G 5N8 (CA); ASHRAF, Salman, Raleigh, NC 27612 (US); MAGNIN, Anthony, A., Willowdale, Ontario M2J 1B4 (CA)
(74) Representative: Browne, Robin Forsythe, Dr.
(86) International application number: PCT/CA94/00514
(87) International publication number: WO 95/08574

(56) References cited:
- EP-A- 0 368 092
- WO-A-87/00177
- GB-A- 2 221 911

## Description

This invention relates to hemoglobin purification by chromatography, and more particularly to chromatographic processes for purifying hemoglobin, on a commercial scale, to obtain a solution in which hemoglobin constitutes at least 99% of the solute. It also relates to novel hemoglobin products having unexpected, beneficial characteristics, for use as a blood substitute.

The development of hemoglobin based blood substitutes continues to command commercial attention, and recent developments have shown that hemoglobin from mammalian blood cells, after suitable modification such as intramolecular crosslinking and in some instances polymerization, shows great promise as the basis of a blood substitute. As development has proceeded, however, the requirements for purity of the hemoglobin have steadily increased. At one time it was believed that hemoglobin simply needed to be stroma free, a condition achieved by washing and gentle lysing of the red blood cells, followed by filtration of the lysate. Subsequently, it was found that the presence of trace residues of impurities such as phospholipids led to more specific reactions, e.g. vasoconstriction, to the product in animal trials. Even after the product has been subjected to several diafiltration steps, it still contains unacceptably high traces of potentially harmful impurities such as erythrocyte enzymes, modified and variant forms of hemoglobin, phospholipids and surface antigens.

One of the most urgent challenges in the blood substitute area, specifically the hemoglobin-based oxygen carriers, is to develop cost effective processes for efficient and economical purification of hemoglobin on a commercial scale. It must compete effectively with other potential blood volume expanders and oxygen carrying fluids proposed for use as blood substitutes.

Whereas resolution and analysis time are important in analytical separations, the critical parameters in preparative chromatography, for commercial or semi-commercial scale use, are:
-- the amount of material isolated per unit time at a specific level of purity (throughput); and
-- economics of the process, such as column sizes, media, buffers, equipment, recycle and re-use of components and reagents and the like.

There are no reported methods for the purification of hemoglobins which meet the criteria for a cost efficient production process.

A hemoglobin-based blood substitute needs to be based either on a single hemoglobin form, or, if more than one form is present, a carefully controlled composition of known hemoglobin forms. Accordingly, a successful hemoglobin purification process needs to be capable of separating one hemoglobin form from another, as well as separating the desired hemoglobin form from contaminating red blood cell such components such as erythrocyte enzymes, proteins, phospholipids and antigens.

Chromatographic methods have been applied to the purification of hemoglobin solutions. U.S. Patent 4.925.474 Hsia et al. describes the application of the techniques of affinity chromatography to hemoglobin purification, using columns in which a ligand showing preferential chemical binding affinity to the DPG site of hemoglobin was bound to the stationary phase of the column.

Ion exchange chromatographic techniques have also been applied to hemoglobin purification. The basic principles of the techniques of ion exchange chromatography are well known. A mixture of different species in a solution is applied to a suitably prepared ion exchange column. Each of the species in the mixture has a different affinity for the chemical reactant groups on the column. By varying the conditions on the column, e.g. the pH of the solution, the individual species can be arranged to bind or to elute from the column selectively, so as to separate one species individually from the mixture. The application of the technique to the purification of proteins such as hemoglobin is economically unattractive, except when used for small scale operations and analytical work. When hemoglobin is to be purified on a manufacturing scale, for use for example as an oxygen carrying resuscitative fluid (blood substitute), the technique, as conventionally applied, is impractical. The amounts of hemoglobin to be absorbed on and subsequently eluted from a chromatography column are so large that the column size requirements become impractically large and expensive.

Christensen et al., J. Biochem. Phys. 17 (1988), 143-154, reported the chromatographic purification of human hemoglobin. The methodology used represented a standard ion exchange chromatographic approach that did not provide opportunities for economical scale-up to production levels.

Winslow and Chapman, "Pilot-Scale Preparation of Hemoglobin Solutions," Methods in Enzymology," Vol. 231, p3 (1994) describe the preparation of stroma free hemoglobin (SFH), highly purified hemoglobin A0 and crosslinked hemoglobin using outdated human blood as starting material. The SFH is prepared by filtration. The Hemoglobin A0 is prepared from SFH by process scale chromatography using a strong anion-exchange medium and an ionic strength gradient.

Both Christensen et.al. and Winslow et.al. report work done at the Letterman Army Institute for Research (LAIR), now known as the Walter Reed Army Institute of Research.

U.S. Patent 5,084,588 Rausch and Feola (Biopure). describes standard anion and cation exchange chromatography methods for application to separation and purification of hemoglobin. In the case of anion exchange chromatography, three standard approaches are listed in this patent:
a) binding of the Hb at elevated pH, and elution with a descending pH gradient or step gradient of lower pH;
b) binding of the Hb at high pH, low ionic strength and elution with a salt gradient;
c) loading under pH conditions where the hemoglobin does not bind to the anion exchanger, but passes through the column unretained, while the impurities (more acidic contaminants) are captured on the column.

Approaches a) and b) have been extensively documented, but are not attractive for large scale production, owing to the limitation of low loading capacities necessary to achieve sufficient resolution of the hemoglobin products. These loading capacities are routinely only 20 -30 mg/ml, and dictate prohibitively large and expensive columns for commercial scale purification of hemoglobin. For example, a single 50 gm dose of final hemoglobin-based oxygen carrier (HBOC) would require a column of 1.5 - 2.5 litres.

Whilst approach c) would appear on the surface to be the most practicable, it turns out in practice that the chromatographic properties of normal human adult, unmodified hemoglobin Ao and some of the major contaminants, such as HbAlc, are not sufficiently distinct for practical application of this approach.

The standard approaches to cation exchange chromatography of mammalian hemoglobin have similar limitations.

U.S. patent 5,084,558 also discloses bovine hemoglobin solutions in which more than 99.9% of the protein present is bovine hemoglobins, but does not present identifying data for the protein constituents..

It is an object of the present invention to provide a novel chromatographic process for the purification of hemoglobin.

It is a further object of the invention to provide a chromatographic process capable of yielding, on a commercial scale and in an economically acceptable fashion, hemoglobin in aqueous solution in a purity in excess of 99%, from a contaminated aqueous solution thereof containing at least 60% of hemoglobin.

It is a further and more specific object of the present invention to provide, in commercial quantities, an aqueous solution of hemoglobin or crosslinked hemoglobin of which the hemoglobin or crosslinked hemoglobin constitutes at least 99% of the solute, and is in a state of purity and, when used as a blood substitute, exhibits properties in vivo, heretofore unreported.

The present invention provides a process whereby an aqueous solution of preselected hemoglobin species, Hb, in which the Hb constitutes from about 60% to about 99% of the dissolved material, is subjected to ion exchange chromatography in two stages. One of the stages is anion exchange chromatography, and the other is cation exchange chromatography. The two types of ion exchange chromatography can be conducted in either order. A crude solution of the preselected hemoglobin species, e.g. HbAo, is fed to the first stage of chromatography, for example anion exchange chromatography, under conditions chosen so that all the constituents of the mixed solute are initially adsorbed on the solid phase of the chromatographic column. For anion exchange this requires a relatively high pH. The conditions are also chosen so that very high effective loading of the column is achieved, using low ionic strength of feed solution, leading to a situation in which substantially all of the accessible sites on the solid chromatographic medium are occupied by species of the feed solution. Eluant collected at this stage is devoid of protein product. Displacement of HbAo and the contaminants having lower affinity for the column under the chosen conditions is then achieved by loading additional volumes of aqueous feed solution as defined above. The condition thereby achieved, herein referred to as an overload condition, causes contaminant species having greater affinity for the column to displace the selected Hb and the contaminant species having lesser affinity for the column, to be eluted from the column. When this first stage chromatographic separation is anion exchange chromatography, contaminants which are more acidic than the selected Hb species are adsorbed on the solid phase of the chromatographic column, and are thereby separated from the selected Hb species, which appears in the eluate along with the more basic contaminants. In the case where cation exchange chromatography is selected as the first stage, the more basic contaminants are separated, by greater affinity to the column, with the more acidic contaminants and the selected hemoglobin species appearing in the eluate.

Then, in the second chromatographic stage of the process of the invention, a second ion exchange chromatographic step is conducted, on the partially purified hemoglobin species contained in the eluate from the first column, under different conditions of pH. When the eluate from the first column contains the hemoglobin species and the more basic constituents, the second stage chromatography is cation exchange chromatography, and visa versa. The feed of this impure solution to the second column is again conducted until saturation loading is reached, and exceeded, so as to create an overload condition in the column. At the overload condition, the preselected hemoglobin species is eluted from the column by displacement effected by the contaminants of greater affinity under the chosen conditions, and the hemoglobin species is thereby collected in the eluate from the second column, in a very high state of purity. The process of the invention may thus generally be called "two stage self-displacement chromatography".

As a result, the Hb species is eluted from the column, in exceptional pure form, normally in excess of 99% purity and in commercially attractive yields. Contaminants more acidic than the selected Hb species remain attached to one column resin, and contaminants more basic than the selected Hb species remain attached to the other column resin. The columns can be easily regenerated using traditional cleaning and regeneration procedures.

An important factor in the successful operation of the process, especially on a large, manufacturing scale, is the selective, high loading of the columns with the impurities, allowing the Hb species to elute.

When the process of the invention is applied to the purification of human hemoglobin A0, there is produced a novel human hemoglobin having a composition and properties hitherto unreported. This hemoglobin product accordingly constitutes another aspect of the present invention, independent from the process of purification described herein. This product has highly desirable properties and characteristics not heretofore seen or reported in connection with any human hemoglobin A0 product, and particularly beneficial in connection with its use as the basis of a blood substitute. Among these properties are the substantially complete absence of viral particles, notably particles of lipid encapsulated viruses; the substantial lack of vasoactivity exhibited by the products when administered in vivo; and lack of toxicity towards endothelial cells upon in vitro incubation, as evidenced by reduced release of lactate dehydrogenase as compared with similar administration of other hemoglobin preparations. In addition, as regards the standard desirable properties of a hemoglobin for use as a blood substitute such as freedom from endotoxins and freedom from other pyrogens, the products of the present invention are also improved in comparison with other, known hemoglobin preparations.

In the accompanying drawings:
FIGURE 1 is a diagrammatic illustration of the initial, anionic exchange portion of the preferred process of the present invention;
FIGURE 2 is a similar illustration of the second, cationic portion of the preferred process of the present invention;
FIGURE 3 is an analytical anion exchange chromatogram derived from the results of Examples 2 and 3 below;
FIGURE 4 is an analytical anionic exchange chromatogram derived from the results of Example 4 below;
FIGURE 5 is an graphical presentation of the results of Example 8 below;
FIGURE 6 is a copy of the paper isoelectric focussing strips of the analysis of the products of Example 13 below;
FIGURE 7 is a copy of the results of the staining and blotting experiments reported in Example 13 below;
FIGURE 8 is a graphical presentation of the results of Example 14 below;
FIGURE 9 and 9A are graphical presentations of the results of Example 15 below; and

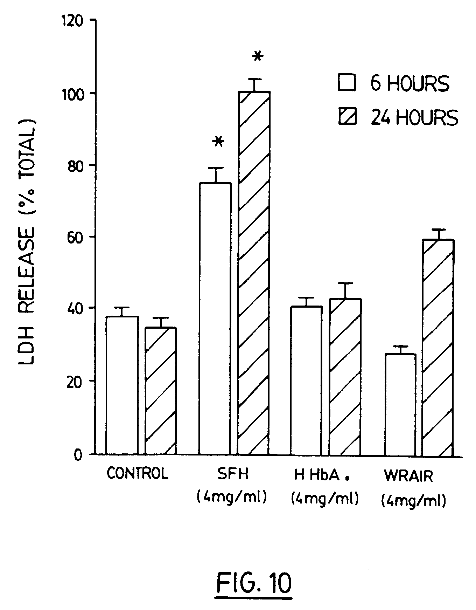

FIGURE 10 is a graphical presentation of the results of Example 16 below.

Displacement chromatography, or displacement elution as it is commonly termed, is a known technique in the field of protein separation. It is one of the three modes by which a sample is recovered from a chromatography column. As applied to separation of respective proteins from a mixture of proteins in solution, proteins of greater affinity will displace proteins of lesser affinity on the matrix, so that a hierarchy of proteins in descending order of affinity is set up within the column, from inlet to outlet. When proteins displace other proteins, a sharp line of demarcation between the species is rarely formed. The application of the technique to prepare highly pure solutions of a single protein such as a hemoglobin is thus contraindicated. If a separate, non-proteinaceous displacing species is chosen, the result may be that the chromatography matrix has bound to it materials having a very high affinity, which is very difficult to regenerate for re-use.

The process shows its chief commercial utility in preparation of purified adult human hemoglobin Ao (HbAo), and so it will be particularly described with reference thereto, for convenience. The process is not, however, restricted to HbAo purification, but is additionally applicable to other preselected hemoglobin species such as selected crosslinked hemoglobin derivatives, selected bovine hemoglobin variants, other hemoglobin variants, such as fetal hemoglobin, sickle cell hemoglobin etc., genetically engineered hemoglobin, etc.

The process of the invention is extremely attractive economically, for use to purify hemoglobin HbAo on a commercial scale to produce blood substitutes. It provides very high effective column loading capacities. It can consequently utilize relatively small size columns, to purify relatively large volumes of impure hemoglobin solution. It can be run batchwise or continuously to purify a given batch of hemoglobin solution as obtained from red blood cells by standard techniques, solutions which normally contain of the order of 80% hemoglobin, but which contain a wide variety of impurities, both proteinaceous and non-proteinaceous. Moreover, the impure hemoglobin solution itself is used as the displacement medium. The provision and use of a separate displacer, with its attendant inconveniences, complications and expense, is thereby avoided.

A typical overall process for making a purified HbAo solution involving the technique of the present invention starts with normal adult, human red blood cells. These are pooled and optionally subjected to filtration to remove leukocytes. The cells are washed to remove serum proteins and lysed to extract the hemoglobin and other cell components. The lysate is filtered to remove red cell membrane and then the resultant hemoglobin solution is diafiltered and concentrated. The impure hemoglobin extract is treated with carbon monoxide to form a CO-Hb complex and heated to pasteurize it, thereby inactivating viral contaminants in the solution. It is then centrifuged and filtered, to remove further remnants and cellular debris. It is now ready to be subjected to the self-displacement process of the present invention.

According to the preferred embodiment of the invention, the first stage of the chromatographic process is the anion exchange process, and the second stage is the cation exchange process. This first, anion exchange process is preferably run at high pH, e.g. pH 7 - 10 and preferably pH 8.5 - 9.0, and under conditions of low ionic strength i.e. low conductivity. The conductivity is suitably less than 3 mS (milli Siemens) and preferably less than 1 mS. Such conditions involve an essentially salt free buffer. The desired HbAo species will initially bind to the column medium under these conditions, along with all other species. As the feed of impure HbAo solution to the column continues, those species which show greater affinity than the HbAo for the matrix, typically the more acidic contaminants, gradually displace the HbAo and more basic contaminants from the column. Eventually, an overload of the column is achieved, so that all the HbAo and more basic contaminants are displaced, leaving only the more acidic contaminants bound thereto. In most cases, this is a loading far in excess of the column manufacturer's recommendations. The chromatographic separation of the more acidic contaminants from HbAo is thus achieved at this stage.

The feed of the impure hemoglobin solution is conducted at a slow linear flow rate, not greater than 10 cm per minute, and preferably at about 1 cm per minute. This permits kinetic equilibration of the column. The feed concentration range is not critical, but is suitably in the range 0.1 - 20%, preferably 2 - 7%.

The achievement of the overload condition can be monitored by analysis of the column effluent - when the composition of the effluent shows none, or only traces, of acidic impurity, the maximum practical loading of the column has been achieved.

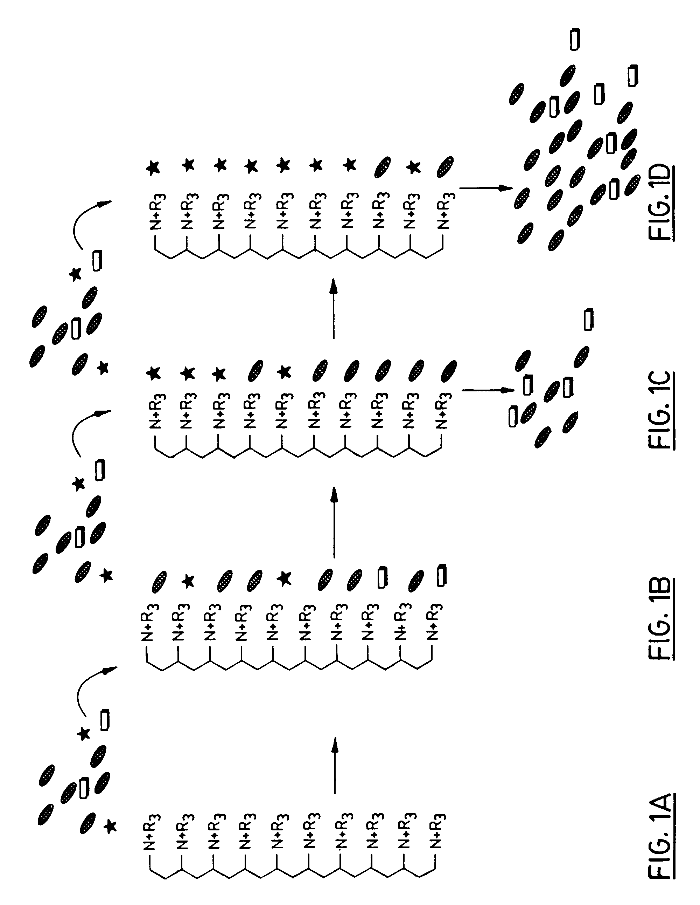

FIG. 1 of the accompanying drawings diagrammatically illustrates the anionic exchange process portion of the present invention. The ion exchange column having chemical groups, -N⁺R₃ (typical of anionic exchange resins, but exemplary only), shown at stage A of the process in Fig. 1 is fed with a solution containing a mixture of species represented by stars, for the more acidic contaminants, ellipses representing HbAo, and rectangles representing the more basic contaminants. At capacity loading, stage B, substantially all the active chemical groups N-R₃ of the column have bound by electrostatic charge to one of the species contained in the mixture.

More of the same solution is fed to the capacity loaded column to reach an overload condition, illustrated in stage C of Fig. 1. Now, all of the more basic contaminants (rectangles) and some of the HbAo (represented by ellipses) are displaced therefrom by the more acidic component (represented by stars), which exhibits the greater affinity for the column under these conditions. Feed of even more of the same solution to the overloaded column continues, to reach stage D illustrated on Fig. 1, at which the acidic species has displaced substantially all of the HbAo from the column, because of its greater affinity under the chosen conditions. Now the effluent issuing from the column contains substantially no detectable amounts of the acidic contaminants. A fairly pure solution of HbAo, but still containing some more basic species (contaminants), is thus obtained.

The second stage of the preferred process of the present invention uses a cationic exchange column, and is fed with eluant from the first column, to a similar overloaded condition. The desired HbAo and other, unwanted, more basic, impurities are bound in overloaded amounts. The partially purified hemoglobin solution from the first column continues to be fed to the second column. Relatively slow feed rates are again employed, suitably 10 cm/min or less, and preferably about 1 cm/min, to permit kinetic equilibration of the solid phase and the liquid phase. The pH of the eluant from the first column is suitably adjusted to the range pH 5-9, preferably pH 6.5 - 8.5, and most preferably pH 7-8, prior to loading of eluant onto the cation exchange column. Low ionic strength of the solution is used, i.e. conductivity less than 3 mS preferably less than 1 mS. Conductivities as large as 2.5 mS are only useful if very slow flow rates and very high feed dilutions are used. Again, overload conditions are used. The proteinaceous contaminants of higher affinity, i.e. those more basic than HbAo, displace the HbAo from the column, and the HbAo is thus eluted and recovered in exceptionally pure form.

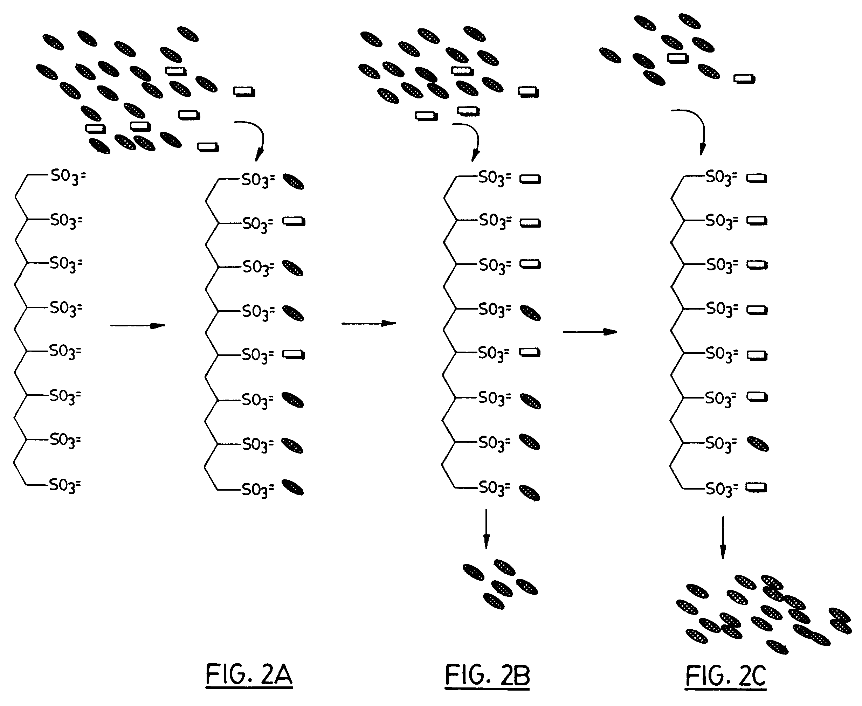

FIG. 2 of the accompanying drawings diagrammatically illustrates, in a manner similar to Fig. 1, the portion of the process taking place in the cation exchange column, shown carrying S03 groups. The eluant from the first column, after suitable pH adjustment, is fed to the column, and initially, in stage A, capacity loading is achieved with both the HbAo (ellipses) and the more basic contaminants (rectangles) binding electrostatically to the chemical groups on the column. Whilst these are illustrated as sulfonic groups, other alternatives such as carboxyl groups could equally well be chosen. Feed of eluent solution to the column continues to achieve an overload condition illustrated in stage B of Fig. 2, in which the more basic contaminant, on account of its greater affinity for the column under these conditions, is replacing the HbAo on the column. By continuing the feed to the overloaded column, as illustrated at stage C on Fig. 2, almost 100% pure HbAo is thus displaced and obtained in solution as the eluent from the column. The higher the column loading accordingly, the higher the percentage recovery of purified HbAo from the column. The eluant is monitored to detect the presence of basic species (rectangles) in the eluant. No further recovery of HbAo by displacement from the column is achieved and maximum loading is defined at that point.

The more basic contaminants, as the term is used herein, refers to those contaminants which elute from the column at a higher pH than that required to elute the HbAo. Conversely, the term "more acidic contaminants" refers to those contaminants which elute from the column at a lower pH than that required to elute the HbAo. In the preferred process according to the invention where anionic exchange chromatography is conducted first, the more basic contaminants elute before HbAo and the more acidic contaminants elute after the HbAo. Depending on the ion exchange media used for the chromatography, different elution profiles can be observed with minor changes in order of elution.

For both the first column and the second column, the flow rates of the impure hemoglobin solution should be optimized for the selected concentration of the impure hemoglobin solution. Linear flow rates of 1 cm/min or less are optimal for hemoglobin solutions at the more concentrated range of 2 - 7%. Flow rates as high as 2 cm/min can be successfully applied to the resolution of more dilute solutions, for example less than 1% Hb. Resolution for ion exchange chromatography at low loading capacities is usually insensitive to linear flow rate; however, for the process of the present invention, kinetic equilibration during chromatography is highly dependent on flow rate. The concentration of the feed, however, is not critical in either stage.

With the displacement chromatography method of the present invention, using firstly anionic exchange chromatography to remove more acidic components and secondly cationic exchange chromatography to remove more basic components, one can achieve high yields of exceptionally pure HbAo, substantially entirely free of detectable amounts of other proteins such as serum proteins and membrane proteins, on a commercial scale. The purities as assayed by analytical anion exchange chromatography are in excess of 99%, and commonly in excess of 99.5%. The effective capacities of the stages are at least an order of magnitude greater than the capacities observed by normal chromatographic approaches. In addition to removal of proteinaceous contaminants, the process of the invention also allows substantially complete removal of modified and other unwanted forms of hemoglobin, to leave substantially pure (>99.5%) HbAo. Moreover, as described in more detail below, substantially complete removal of encapsulated viral particles, active or inactivated, can also be achieved.

The scale-up advantages of the process of the present invention, as compared with a process using standard ion exchange adsorption/elution chromatography, are demonstrated in the following Table 1. The figures in the "Self Displacement" column are derived from the working examples of the process of the present invention, described below. Those in the column "Adsorption/Elution" are obtained from the aforementioned Winslow et al. paper.

**TABLE 1**

| **OPERATING PARAMETERS FOR HEMOGLOBIN AO PURIFICATION USING ADSORPTION/ELUTION CHROMATOGRAPHY * vs SELF DISPLACEMENT CHROMATOGRAPHY** | | |
|---|---|---|
| | SELF DISPLACEMENT | ADSORPTION/ ELUTION |
| Column Volume | 5 and 3 L | 120 L |
| Column Capacity | 200-300 mg/ml | 15 mg/ml |
| g Hb Loaded | 1234 | 1818 |
| % Recovery | 81 | 55 |
| g Hb Collected | 1000 | 1000 |
| Buffer Volume For Run and | 132 L | 900 L |

| | | |
|---|---|---|
| *Winslow and Chapman, op. cit. | | |

It is clear from the these figures that the self-displacement chromatography process of the present invention can be run faster, with reasonably small columns. It can be run under low pressure conditions and at ambient temperatures. Effective loading capacities which are 10 - 20 times the capacities reported for conventional chromatographic processes can be attained, making possible the use of relatively small column volumes. This leads to reductions in water requirements, reduced waste disposal and reduced buffer requirements, all of which improve significantly the commercial economics of the process.

The purified hemoglobin solution constituting the eluant from the cationic exchange column can be diafiltered into a buffer of choice, and adjusted to the desired HbAo concentration.

There is nothing particularly critical about the ion exchange gel material used in the process of the present invention as the stationery phase. The choice is well within the skill of the art. There can be used any of the common, commercially available such gels, provided that they can be successfully derivatized to operate in an ion exchange mode under the selected affinity conditions for the species to be separated. General classes of supports for ion exchange media include macroporous, macroreticular and non-porous types, divinylbenzene-crosslinked polystyrenes or polymethacrylates, polyalkyleneamines, cellulose, dextran, agarose, silica, ceramic, glass, alumina and metallic particles and others. Commercially available supports include those sold under the trade-marks POROS media, Fractogel, HyperD, Dowex, Amberlite, Duolite, Bio-Rex, Chelex, Sephadex, Sepharose, Toyopearl, Macro-prep, Bio-protocol, Accell, Mono types and others. Functional groups attached to such supports for the purpose of ion exchange include sulphonate, sulphopropyl, carboxymethyl, carboxylate, phospho, quaternary amine, quaternary aminoethyl, polyethyleeneimine, trimethylaminoethyl, diethylaminoethyl, dimethylaminoethyl, aspartamide and others.

The process of the invention can also be operated to produce an HbAo solution of at least 99.5% purity, and, unexpectedly, free from viral particles, active or inactivated, especially lipid encapsulated viruses such as HIV, herpes simplex virus (HSV) and bovine diarrhea virus, which in many instances is a model for human hepatitis C virus. These are some of the most serious and troublesome viruses contaminating blood. This is a very significant, added advantage to be derived from the use of the present invention, and one which could not have been predicted before the process was tried and the results analyzed. While preferred processes according to the invention involve a step of pasteurization to inactivate viruses, prior to purification, as described above, the achievement of viral particle removal as part of the purification process is especially advantageous. It removes from the blood substitute material any active viral particles which may have survived the pasteurization, and any residual inactivated viral particles. All this can be achieved without the use of any additional, specific viral particle removal step, and without the addition of any special reagents or the like for the purpose of viral removal. Substantially complete removal of lipid encapsulated virus can be achieved. A significant reduction in the quantity of other viruses is also achieved.

The hemoglobin products of the present invention have a higher degree of purity than any previously reported hemoglobin products, as far as the applicants are aware. Perhaps due to their exceptional state of purity, or perhaps due to other, as yet not fully explained factors, the products of the invention exhibit unexpected and highly advantageous properties not previously reported for hemoglobin products, at least for those produced on a large scale.

More specifically, the products of the present invention, HbAo, are free of all red blood cell membrane proteins as determined by isoelectric focussing and immunoblot analysis. Trace amounts of human serum albumin (HSA), at levels less than 4 µg and typically from 2-4 µg HSA/100 mg Hb are present as detected by immunoabsorption techniques. The only other detectable proteins sometimes found in the products of the invention are fragments of the α and β globin chains of hemoglobin, and these do not exceed 1 µg per 100 mg hemoglobin. At this extremely high level of purity, the product of the present invention is unique, and demonstrates the unexpected properties described below. One of these is reduced or absent vasoconstrictor activity. Another is lack of toxicity towards cultured endothelial cells.

Highly purified hemoglobins reported previously, both crosslinked and uncrosslinked, have resulted in pressor responses upon infusion ^{(1,2)}. Since the products were apparently so highly purified, current theories propose that there must be an inherent property in hemoglobin itself, which is responsible for this. Specifically, it has been thought that it is the ability of hemoglobin to bind nitric oxide that is responsible for this effect. Nitric oxide, also referred to as endothelium derived relaxing factor, is a potent vasodilator, synthesized by endothelial cells through the intermediary of nitric oxide synthase. Since hemoglobin molecules will bind nitric oxide with high affinity, it has been postulated, and is currently accepted, that intravenous infusion of hemoglobin solutions, especially solutions of non-crosslinked hemoglobin, will sequester nitric oxide and induce a hypertensive response, with vasoconstriction.

The finding that products of the present invention are devoid of vasopressor activity, in models of isovolemic exchange and hemorrhagic shock, is thus completely unexpected, and is contrary to current theories and beliefs relating to intrinsic properties of hemoglobin. The present invention suggests that it is not wholly an intrinsic property of hemoglobin which is responsible for its previously reported vasoconstrictive activity, but at least in part it is due to an impurity which is present in extremely small amounts and which has not previously been successfully removed, despite the reports of extremely high purity levels of previously reported hemoglobins.

The benefits of absence of vasopressor activity of the products of the present invention persist in crosslinked and polymerized hemoglobin products made from the products of the present invention, e.g. using the process described in commonly assigned U.S. Patent application serial no. 08/231,945 filed April 21, 1994.

In addition, products of the present invention exhibit a greatly reduced toxicity towards endothelial cells, upon in vitro administration, than previously reported hemoglobins. Studies with previously described oxyhemoglobin solutions, including chromatographically purified hemoglobins, have demonstrated cellular injury, as indicated by significant increases in lactate dehydrogenase to an endothelial cell culture medium, relative to levels of lactate dehydrogenase in medium of control endothelial cell cultures. Lactate dehydrogenase is a marker of cellular injury. This toxicity again has been postulated and accepted to be an intrinsic property of oxyhemoglobin, because the test solutions contained oxyhemoglobin in a highly purified condition.⁽³⁾

Unexpectedly, and significantly, it has been found that hemoglobin according to the present invention does not mediate release of lactate dehydrogenase. In contrast to hemoglobin of the present invention, significant increases in lactate dehydrogenase release were observed upon incubation of endothelial cells with stroma-free hemoglobin solutions purified by other chromatographic processes.

It is generally accepted, and indeed it is self-evident, that any blood substitute and any ingredient of or raw material for a blood substitute, should be sufficiently purified so that it is free from contaminants known to be harmful. Previously recognized such harmful contaminants include cell stroma, endotoxins and other pyrogens, non-hemoglobin proteins, phospholipids, etc. Prior art blood substitutes have been reported to be "substantially free" of one or more such contaminants. The term "substantially free," however, is a relative, subjective term, the interpretation and limitation on which depends on the manner of determination of the relevant parameter used and described in the relevant prior art item. Improved purification and analysis techniques have lead to reports of blood substitutes and hemoglobin products of higher and higher levels of purity. For example, the prior art literature contains reports of hemoglobin solutions in which more than 99.9% of the protein present is hemoglobin, in one form or another (see aforementioned U.S. patent 5,084,588 Rausch et al). It is postulated, in accordance with the present invention, that even this purity is not high enough for a successful blood substitute, because the nature of the residual impurity may be causing vasoconstrictive effects and exhibiting cellular toxicity.

Stroma free hemoglobin as available from the Walter Reed Army Research Institute (WRAIR) and generally recognized as the industry standard for purity, can be shown to contain a large number (>10) different, contaminating proteins, many of which are present at less than 0.01% of the total protein. Whilst this is a very small amount superficially, the fact is that hemoglobin as a blood substitute is administered to a subject in relatively large quantities, and it is the total amount of contaminant administered which determines whether or not it is harmful, not the concentration of it in any administered mixture. A single unit of a hemoglobin based blood substitute solution will contain from 25 - 50 grams of hemoglobin, so that 0.01% impurity represents 2.5 - 5 milligrams of impurity. Among the wide variety of proteins present, there may well be one or two which are extremely harmful at this level. The present invention provides a hemoglobin product which has a non-hemoglobin protein content below 0.5% and preferably below 0.1% and presumably but not necessarily as a consequence shows no discernible vasoconstrictive effects and no significant toxicity towards endothelial cells. The invention also provides a process by which such hemoglobin solutions can be prepared, on a commercially attractive scale, as discussed above.

Yet another significant advantage of the products of the present invention is their reduced procoagulant activity. It appears that the products are very significantly reduced in content of agents which potentiate the coagulation of blood, as compared with other stroma-free hemoglobin products available and previously reported.

The invention is further described, for illustrative purposes, by the following, non-limiting examples.

### EXAMPLE 1 - PREPARATION OF CRUDE Hb LYSATE

Ninety-six units (approx. 30L) of packed red blood cells (RBCs) of the same blood type were pooled, and then diluted with 24 L of 0.9% saline solution. This pool was then filtered through 20µ and 8µ polypropylene filters to remove any residual leukocytes.

After leukocyte filtration, the RBCs were washed with 6 volumes of 0.9% saline in a constant volume diafiltration using 3.5m² of 0.3µ Sepracor polyethersulphone hollow fibre membranes. The washing buffer was then replaced with 50mM Tris lysing buffer, and the RBCs were gradually lysed into 6 volumes of this lysing buffer.

The crude lysate was collected and concentrated from approximately 2.5% Total Hemoglobin (THb) to approximately 9.0% THb using a 30K Molecular Weight Cut Off (MWCO) Millipore PTTK membrane. Once concentrated, the tank was charged with CO gas to convert the hemoglobin to COHb form. The lysate was pasteurized at 62 ± 2°C for 10 hours in a jacketed tank. The pasteurized lysate was cooled and then centrifuged. Further depth filtration through Millipore 0.8µ filters prepared the pasteurized lysate for diafiltration on another Millipore 30K PTTK membrane. The material was diafiltered with 5 mM Tris, pH 8.9 until its conductivity was < 0.3 mS and the pH was 8.9 ± 0.2. It was then diluted to 4.5 - 5% THb, at which point it was ready for subsequent chromatographic purification.

### EXAMPLE 2 - SELF-DISPLACEMENT CHROMATOGRAPHY ON ANION EXCHANGE RESIN

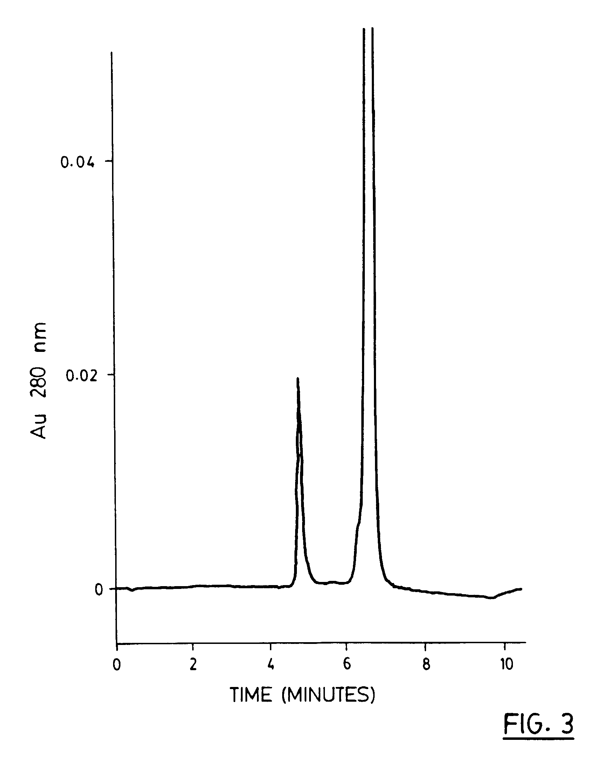

A 1 x 10 cm column packed with the anionic exchange resin PerSeptive Poros HQ-50 was washed with 4 column volumes of 1N NaCl and equilibrated with 5 mM Tris buffer, pH 8.8. After equilibration 50 ml of a 3.0% (3 g/100ml) crude hemoglobin lysate (∼85% HbAo) in 5 mM Tris, prepared as described in Example 1, pH 8.8, was loaded onto the column at 0.78 ml/min (1 cm/min). This led to an eventual overload of the column - 200 mg of the solute loaded per ml of resin, compared with the manufacturer's stated loading capacity of 50 mg/ml. The eluant was collected. The column was then washed with 2 column volumes of 5 mM Tris, pH 8.8 buffer and this eluant was pooled with eluant collected during loading. The column was then washed with 1N NaCl to elute the retained proteins, and this wash eluant was discarded. The column was regenerated using 3 column volume 1M NaCl/HCl and 4 column volume 1 M NaOH. The hemoglobin eluant from the anion-exchange resin was analyzed on an analytical anion-exchange column using a pH gradient, as described in more detail below, in Example 11. It was determined to be about 95 - 96% HbAo, as shown on Fig. 3, with recovery of 90% of the HbAo loaded on to the column.

### EXAMPLE 3 - SELF-DISPLACEMENT CHROMATOGRAPHY ON ANION EXCHANGE RESIN

A 1 x 10 cm column packed with Merck Fractogel-TMAE was washed with 4 column volumes of 1N NaCl and equilibrated with 5 mM Tris buffer, pH 8.8. After equilibration, 50 ml of a 3.0% (3 g/100 ml) crude hemoglobin lysate (-85% HbAo) in 5 mM Tris, prepared as described in Example 1, pH 8.8, was loaded onto the column at 0.78 mL/min (1 cm/min). This similarly led to an eventual overload of the column. The eluant was collected. The column was then washed with 2 column volumes of 5 mM Tris, pH 8.8 buffer and this eluant was pooled with eluant collected during loading. The column was then washed with 1N NaCl to elute the retained proteins, and the wash eluant was discarded. The column was regenerated using 3 column volume 0.5 N HCl and 4 column volume 1 M NaOH. The hemoglobin eluant from the anion-exchange resin was analyzed on an analytical anion-exchange column using a pH gradient, as described in more detail below in Example 11, determined to be about 95-96% HbAo, as shown in Fig. 3, with a recovery of 73% of the HbAo loaded on to the column.

### EXAMPLE 4 - SELF-DISPLACEMENT CHROMATOGRAPHY ON A CATION-EXCHANGE RESIN

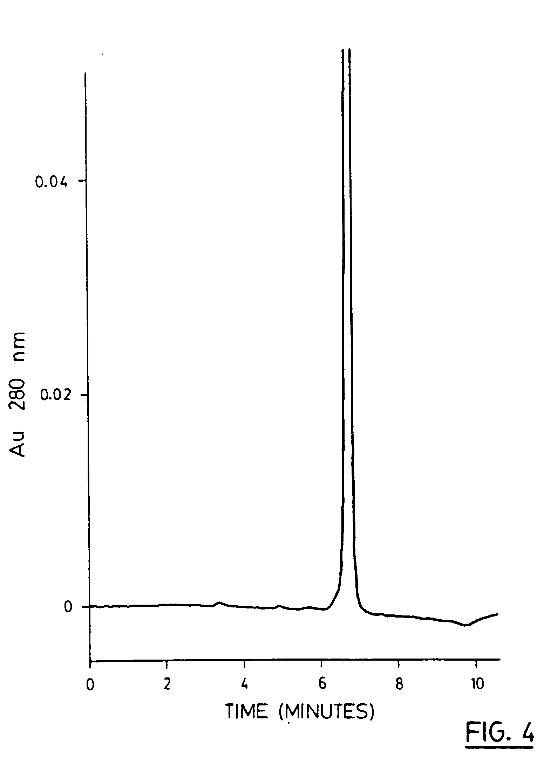

A 1 x 10 cm column packed with Perseptive Poros HS-50 was washed with 4 column volumes of 1N NaCl and equilibrated with 5 mM Tris buffer, pH 7.5. After equilibration 120 ml of a 2.0% (2 g/100 ml) anion-exchange purified hemoglobin ∼95% HbAo) in 5 mM Tris, pH 7.5, prepared as described in Examples 2 and 3, was loaded onto the column at 0.78 ml/min (1 cm/min). This led eventually to a substantial overload of the column, to the extent of about 5 times the manufacturer's recommendation. The eluant was collected. The column was then washed with 2 column volumes of 5 mM Tris, pH 7.5 buffer and this eluant was pooled with eluant collected during loading. The column was then washed with 1N NaCl to elute the retained proteins, and this was discarded. The column was regenerated using 3 column volumes 0.5 N HCl and 4 column volumes 1 M NaOH. The hemoglobin eluant from the anion-exchange resin was analyzed on an analytical anion-exchange column using a pH gradient, and was determined to be > 99% HbAo, as shown on Fig. 4, with a recovery of about 90% of the HbAo loaded on to the column.

### EXAMPLE 5

The effect of conductivity on the self-displacement chromatography on an anion-exchange resin (first stage) was studied.

Effective displacement chromatography is critically dependent on the ionic strength of buffer and/or sample, as determined by conductivity. The experiment summarized below in Table 2 was performed feeding crude lysate of Example 1 to the Poros HQ-50 anion exchange resin from PerSeptive Biosystems Inc. The eluant from the column was analyzed by analytical anionic exchange chromatography. The fractions obtained during run no. 1 were all free from detectable acidic contaminants, whereas even the first fraction from run 2 was contaminated with acidic contaminants.

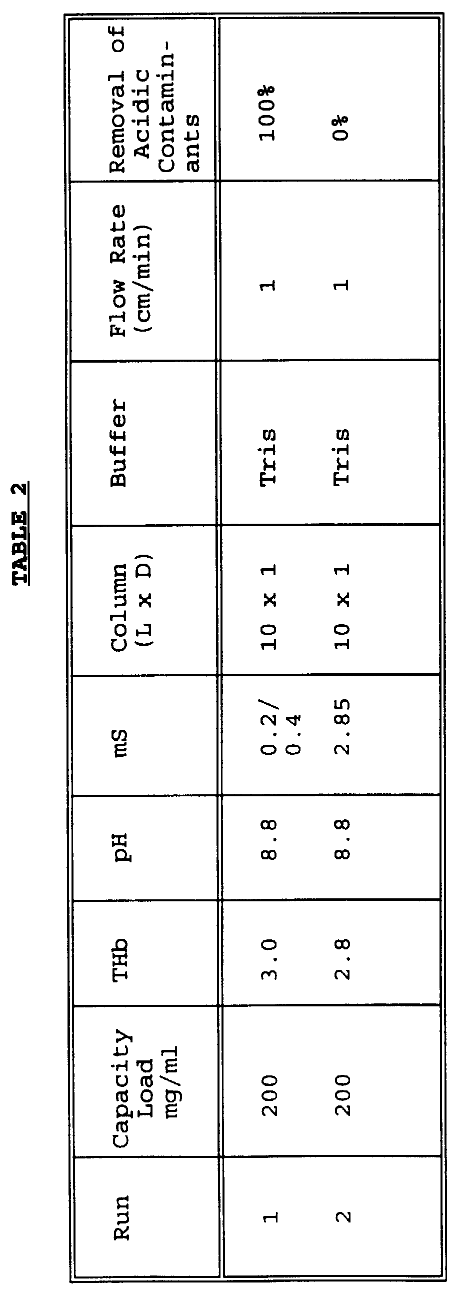

### EXAMPLE 6 - YIELD AS A FUNCTION OF PROTEIN LOAD.

Overloading the column achieves a higher recovery of purified HbAo. The experiments reported below in Table 3 demonstrate a very high overload on a 10 ml column of cationic exchange Poros HS-50 resin, which ensures all of the binding sites are occupied by the contaminants, allowing a higher recovery of purified HbAo. The overload in the first experiment was 13.5 times the manufacturer's recommendation (308 mg protein/mL). That in the second experiment was about 5 times the manufacturer's recommendation. The higher overloading leads to a significant improvement in recovery of HbAo from the column.

**TABLE 3**

| Load (mg Protein mL | THb | pH | mS | Column (LxD) | Buffer | Flow Rate cm/min | Freedom From Basic Contaminants |
|---|---|---|---|---|---|---|---|
| 308 | 3.1 | 7.5 | 0.5 | 9.5xl | Tris | 0.6 | 100% 88% Recovery |
| | | | | | | | |
| 216 | 2.9 | 7.4 | 0.6 | 9.5x1 | Tris | 0.6 | 100% 77% Recovery |

### EXAMPLE 7 - COMPARISON BETWEEN SMALL AND LARGE COLUMNS

The displacement chromatography process of the present invention can be used on large and small columns. Using anionic exchange resin Poros HQ-50 and cationic exchange resin Poros HS-50 sequentially, this comparison was done utilizing 15 and 10 litre columns, respectively, with 10 ml columns. The feed solution to the Poros HQ-50 column was prepared as described in Example 1. The eluant from the HQ-50 column was fed to the HS-50 column. Table 4 shows the conditions and results of the use of the Poros HQ-50 columns. Table 5 shows the results of the use of the POROS HS-50 columns. The results show that both stages of the process can be scaled up to at least 10-15 litre column size, for commercialization.

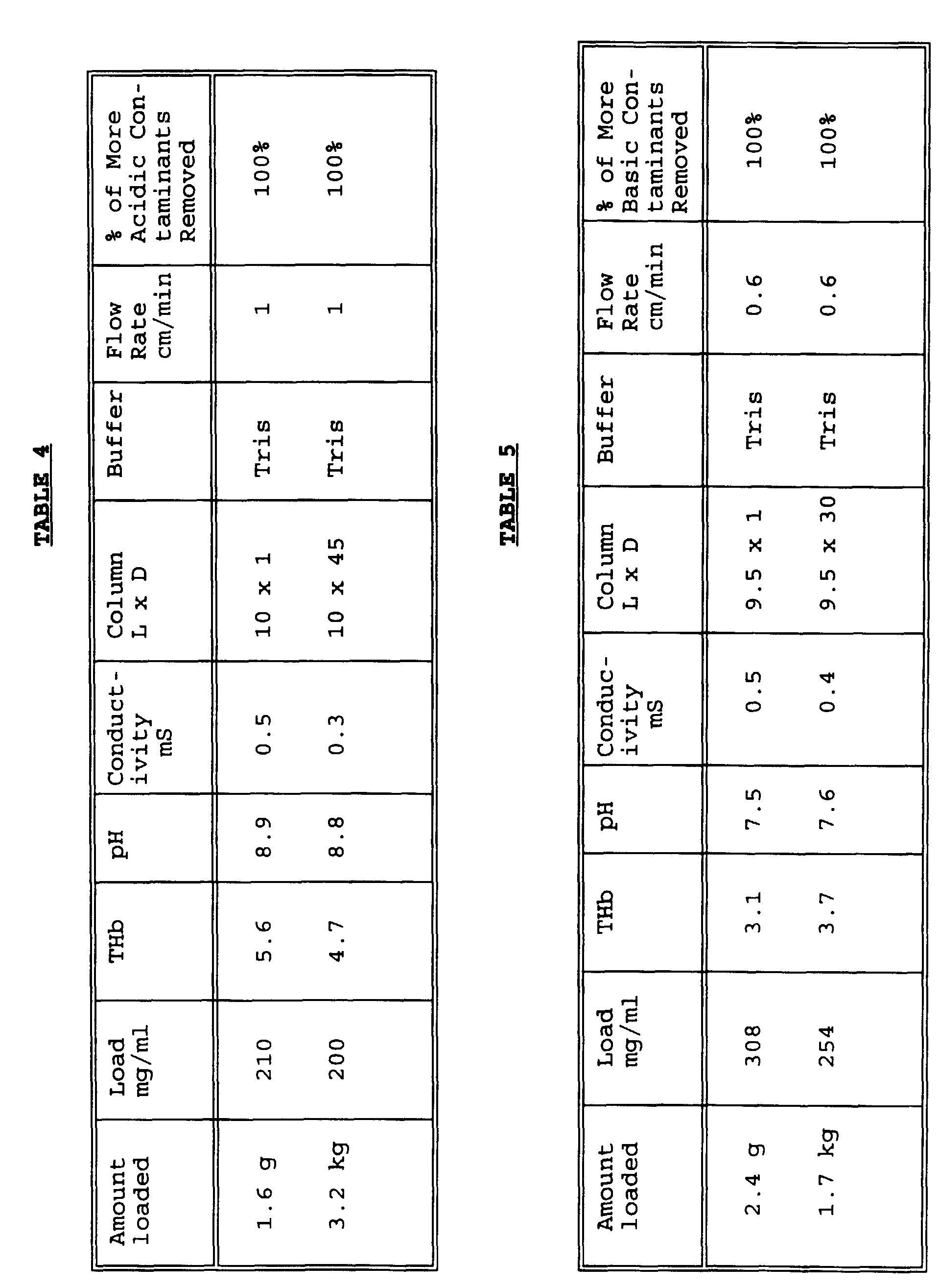

### EXAMPLE 8 - ANALYSIS OF CRUDE HEMOGLOBIN LYSATE AND PURIFIED HEMOGLOBIN BY ISOELECTRIC FOCUSING (IEF)

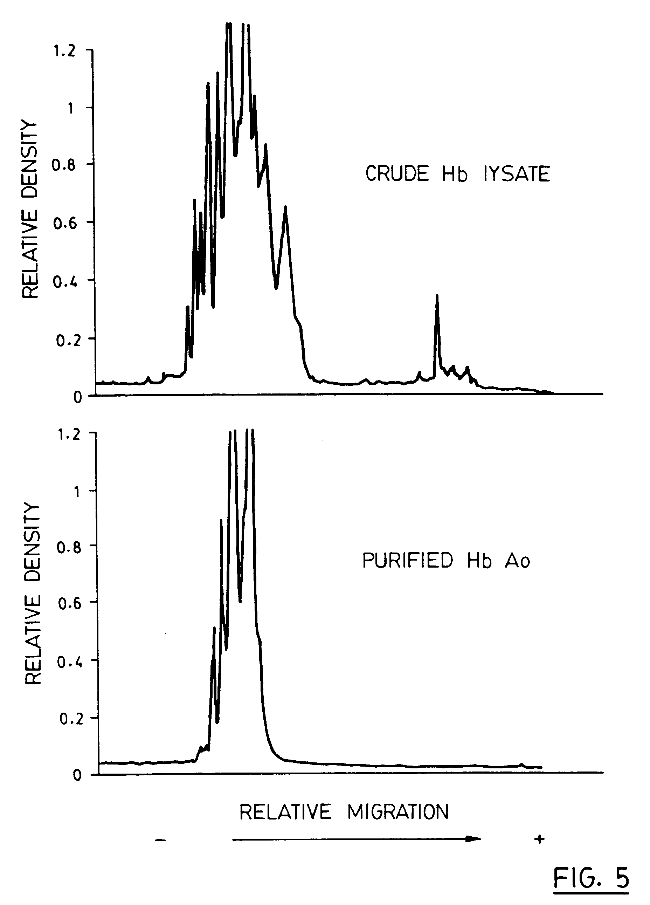

Isoelectric focusing (IEF) analysis was performed using an agarose based gel into which a stable pH gradient was established using commercially available ampholytes. The ampholytes used were such that 90% of them were in the pH range of 5-8 and 10% were in the pH range of 3.5-10. The agarose in the gel was at a final concentration of 1.25% and the ampholytes were present at a final concentration of about 2%. For the purpose of assessing purity, the gels were grossly overloaded (1 mg protein was loaded per lane). Electrophoresis was performed at low current settings to ensure that the proteins migrate as a uniformly straight band. After a steady state was reached, the voltage and the current was increased for a short period to sharpen the bands further. After electrophoresis, the proteins in the agarose gel were fixed using trichloroacetic acid and sulfosalicylic acid solution. After the fixation step, the gel was stained with Comasie-Blue to visualize the protein bands. For scanning purposes, the gel was air dried, and then a laser densitometer was used to obtain tracing of the electrofocussed lanes containing crude hemoglobin lysate and purified hemoglobin. Figure 5 shows a comparison between the IEF of crude hemoglobin lysate, prepared as described in Example 1, and purified HbAo prepared according to the invention. Purification of the hemoglobin solution was conducted using sequentially a Poros HQ-50 column as described in Example 2, and a Poros HS-50 column as described in Example 4.

### EXAMPLE 9 - IMMUNO-ANALYSIS OF CRUDE HEMOGLOBIN LYSATE AND PURIFIED HEMOGLOBIN

To analyze the amount of various contaminants in the crude hemoglobin lysate from Example 1 and purified hemoglobin, prepared according to the process of the invention, the technique of immunostaining was employed. After the electrofocussing of proteins on the agarose gel, the proteins were transferred onto a nitrocellulose paper using capillary blotting. The remaining free binding sites on the paper were blocked by incubating the blot in a hydrolyzed fish gelatin solution. After incubation, the blot was washed with tris-buffered saline (TBS) and then incubated with the primary antibody. (The primary antibody is the antibody against which the sample is to be tested, e.g. anti-human serum albumin (anti-HSA) would be used to test for HSA in the hemoglobin solutions). After this incubation, the blot was washed with TBS and then incubated with the secondary antibody, conjugated to a marker enzyme (e.g. Horse radish peroxidase), which is antibody against the primary antibody. After incubation with the secondary antibody, the blot is washed with TBS again, and then a substrate for the marker enzyme is added. A coloured precipitate will appear where the secondary antibody has bound to the primary antibody, which has bound to its antigen, thus indicating the amount and position of the antigen to the primary antigen on the IEF gel.

The results are shown in Table 6 below, on which "++++++" indicates a strong signal, "+" indicates a barely detectable signal, and "-" denotes no signal detected.

**TABLE 6**

| **ANTIBODY USED** | **CRUDE HB LYSATE** | **HEMOGLOBIN** |
|---|---|---|
| Human Serum Albumin | +++++++++++ | +^{a} |
| Red Blood Cell (RBC) | +++++++ | +^{b} |
| RBC Membrane | +++++ | - |
| Human Plasma | +++++++++++ | +^{c} |
| Carbonic Anhydrase I | +++ | - |
| Spectrin | ++++ | - |
| Glycophorin | ++ | - |

| | | |
|---|---|---|
| a. Using HSA standards on the immunoblots, the amount of HSA present in the purified HbAo was estimated at <50ng/mg Hb (<50 ppm). | | |
| b. A trace contaminant with a pI ∼5.2 was detected which cross-reacts with anti-RBC antibody. Other researchers have also reported a similar protein in their purified Hb solutions - see Christensen et al., op. cit. | | |
| c. Anti-HSA antibody present in the anti-human plasma detected the HSA present in the purified HbAo. | | |

### EXAMPLE 10 - HUMAN SERUM ALBUMIN - IMMUNO DETECTION (HSA-ID)

A chromatographic method was used to quantitate the amount of HSA present in the hemoglobin solutions. The method used an antibody (anti-HSA) covalently bound to a matrix in a column. When sample is injected onto the column, everything but the antigen for the antibody (HSA) passes through during the load/wash step. Upon using the elute buffer the bound antigen (HSA) elutes from the column and the area of the peak is used to quantitate the antigen present in the sample.

The ImmunoDetection* (ID) cartridge used for the assay was from PerSeptive Biosystems. The flow rate used for the assay was 2 ml/min. The cartridge-shaped column (1.6 mmD x 26 mmL) was equilibrated with the loading buffer (10 mM phosphate buffer + 300 mM NaCl, pH 7.2), and then 50 µl of the sample was injected. The loading buffer was used to wash the column for 0.25 minutes, and then the elution buffer was used (for 3.5 minutes) to elute the bound HSA from the column. The eluant was monitored using a Beckman diode array detector set at 220 nm and 414 nm. The column was then re-equilibrated with the loading buffer for the next injection. Using this method, the amount of HSA present in the purified hemoglobin was estimated to be not greater than 0.0005% weight, based on hemoglobin.
* Trademark

### EXAMPLE 11 - ANALYTICAL ANION EXCHANGE CHROMATOGRAPHY

The chromatographic method used to analyze the purity of hemoglobin in the products of Examples 2 and 4, was as follows. The assay was based on a method reported by Huisman & Dozy⁽⁴⁾, loading sample on to an anion-exchange column at high pH (pH 8.5), such that all the protein bound to the column, and then a pH gradient from pH 8.5 to pH 6.5 was used to sequentially elute the proteins from the column. Using this chromatographic assay, very similar proteins (e.g. various hemoglobin variants) could be separated. A 4.6 mm x 100 mm analytical anion exchange column from PerSeptive Biosystems was used for the assay. The buffers used were A) 25 mM Tris + 25 mM Bis-tris, pH 8.5 and B) 25 mM Tris + 25 mM Bis-tris, pH 6.5. The assay was performed at a flow rate of 5 ml/min. After the column was equilibrated with buffer B, 10 µl sample (conc. 10 mg THb/ml) was injected on to the column, and the gradient (100% buffer A to 100% buffer B over 25 column volumes) was started. Eluant was monitored by UV detection at 280 nm to identify protein peaks. Figure 3 shows the chromatographic profile of anion-exchange purified hemoglobin (Example 2 product) and Figure 4 shows the purified HbAo (Example 4 product) when analyzed by this assay.

This method is a standard assay for checking the purity of hemoglobin solutions. The purified HbAo of the invention was found to be free of all contaminants observed in stroma-free hemoglobin. A small shoulder preceding the HbAo peak was observed occasionally, attributable to the oxidized form of HbAo (MetHbAo). The purity of this invention is routinely found to be greater than 99.5% by this analytical method.

### EXAMPLE 12 - VIRUS REMOVAL

The process of the present invention was tested for its ability to remove viruses from the crude hemoglobin

To samples of a crude lysate prepared as described in Example 1, and to samples of partially purified hemoglobin solution issuing from the anion column, i.e. the product from Examples 2 and 3, test quantities of the following viruses were added, in active form:
Poliovirus Type 1
Human Immunodeficiency (HIV)
Bovine Diarrhea Virus (which is a model for the human hepatitis virus)
Herpes Simplex Virus Type 1 (HSV-1).

Aliquots of the eluants from the respective columns were added to test cell systems. In the cases of poliovirus, herpes virus and bovine diarrhea virus, the active virus present interacts with test cells to give plaques. Known, standard assays were conducted. In the case of HIV, the aliquots were added to tissue culture media. The amount of virus present was determined by a change in the growth of the cell line. This change is known as TCID₅₀ (tissue culture infectious dose endpoint at 50%). The results are reported in Table 7 below.

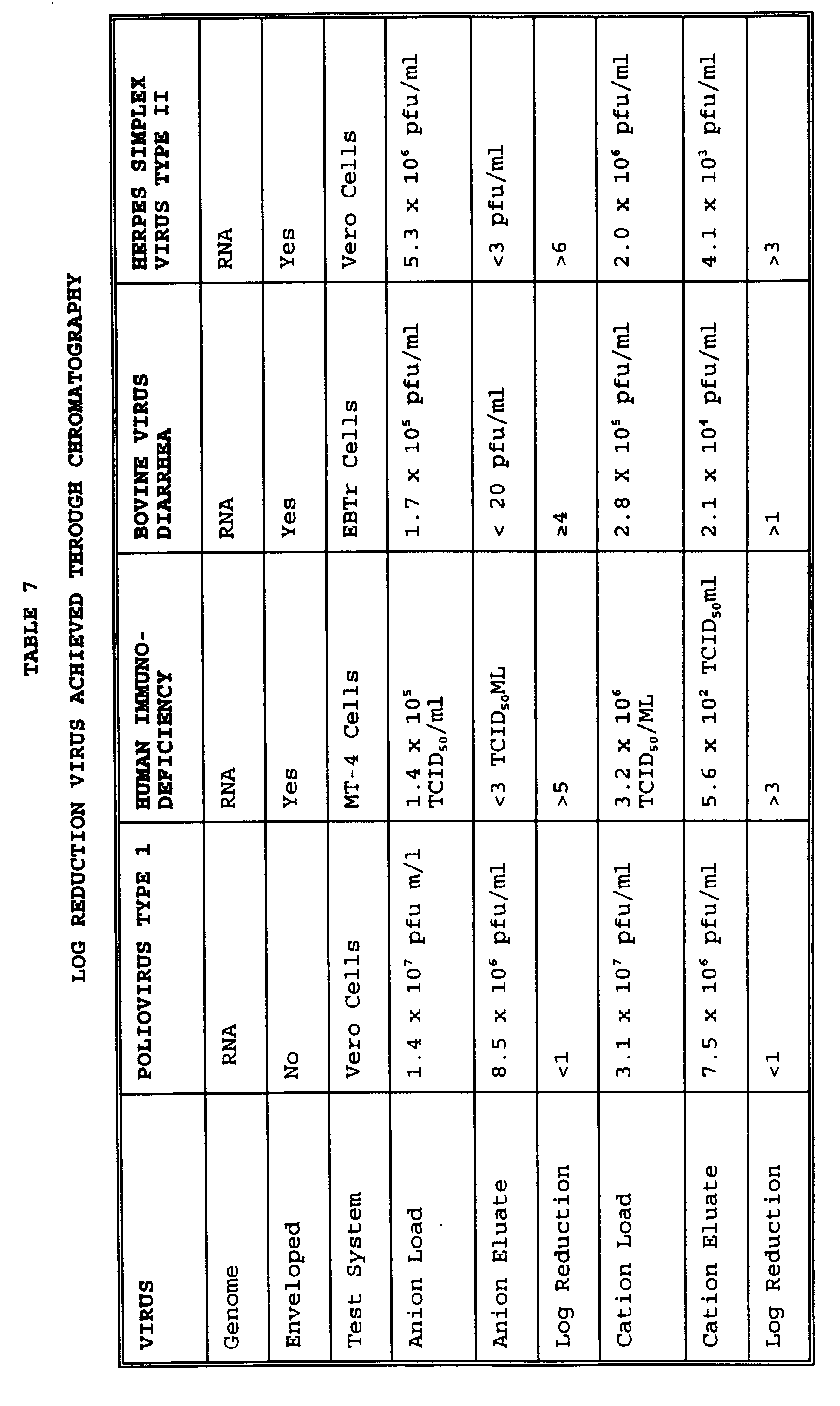

These results show a small reduction in the amount of polio virus, using either the anionic exchange self-displacement or the cationic exchange self-displacement steps of the present invention, but remarkable reductions in virus load in the cases of HIV, HSV and bovine diarrhea, following anion exchange chromatography and to a lesser extent following cation exchange chromatography. This was completely unexpected, but presents a significant advantage of the use of the present invention.

### EXAMPLE 13 - PURITY ANALYSIS BY ISOELECTRIC FOCUSSING AND WESTERN BLOTTING

Isoelectric focussing (IEF) experiments were performed using the technique of Saravis and Zamcheck. ⁽⁵⁾

IEF gels were cast using agarose (1% final concentration) and the pH gradient was created using ampholytes (Pharmacia Biotech Inc., Piscataway, N.J.) such that 90% of the ampholytes were in the pH range of 5-8, and 10% were in the pH range of 3.5-10. Typically 10 µl of 100 mg/ml Hb solutions were loaded onto the IEF gel. After focussing the proteins, the gel was fixed using 10% trichloroacetic acid (TCA), dried, and stained with Coomassie-Blue [3]. Normally, the total protein load/lane was 1 mg.

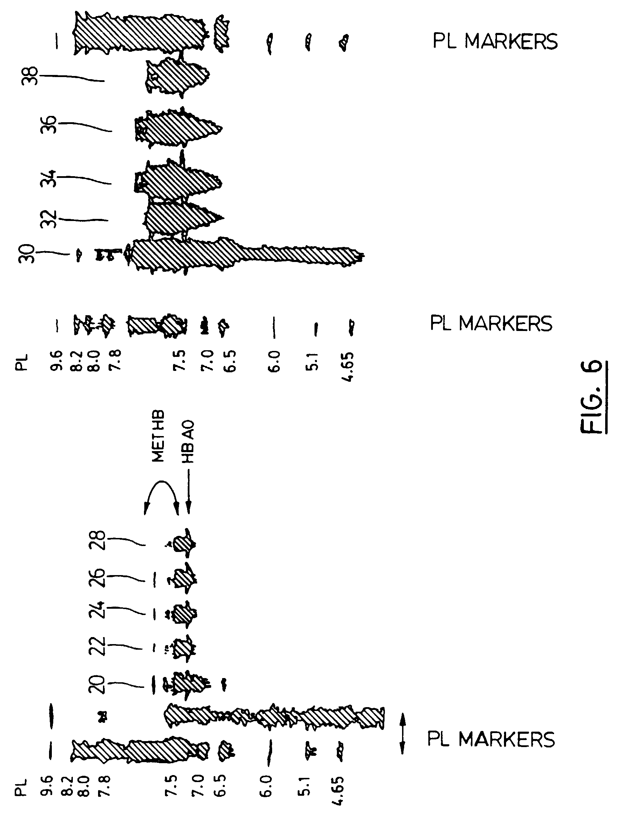

Isoelectric focussing (IEF) analysis was performed to compare the purity of SFH with four (4) different lots of purified HbAo according to the invention. 50 µg total Hb or 1 mg total Hb were loaded per lane. The results are shown in Fig. 6. Lane 20 represents stroma-free hemoglobin at 50 µg total Hb loading, and lanes 22, 24, 26 and 28 represent lots of purified Hb according to the invention, at the same loading. Lane 30 represents SFH at 1 mg total loading, and lanes 32, 34, 36 and 38 represent the lots of purified Hb according to the invention, at the same loading. The 50 µg total Hb sample loading shows the purified HbAo and the SFH to be of similar purity. However, at 1 mg total Hb/lane numerous red blood cell proteins, either plasma proteins or membrane proteins, are observed in the SFH lane that are absent in the purified HbAo lane. A very faint protein band of pI around 5.2 was observed in the purified HbAo lane, probably due to globin chain fragments. The bands above the HbAo band in the 50 µg sample load lanes are due to the more positively charged MetHb species, that form during the electrofocussing.

After the isoelectric focussing, Western blotting, which involves the use of antibody staining (immuno-staining) was employed, further to scrutinize the purity of the product of the invention. Thus, the proteins from the IEF gel were transferred to nitrocellulose paper using capillary blotting. The remaining free sites on the nitrocellulose paper were blocked by incubating the blot with hydrolyzed fish gelatin (Hipure Liquid Gelatin, Norland Products Inc., New Brunswick, N.J.). The blot was then incubated with rabbit antibody to human plasma (Dako Corp., Santa Barbara, CA, lot #010) (antibodies diluted 1:500 in Tris-buffered saline). After overnight incubation, the blot was washed with Tris-buffered Saline (TBS) containing 1% fish gelatin, and then incubated with goat-anti-rabbit IgG-Horseradish Peroxidase (HRP) (diluted 1:1000 in TBS). After incubation for one hour, the blot was washed again with TBS containing 1% fish gelatin. The antigen-antibody-HRP complex was visualized by using 4-chloro-1-naphthol (30 mg/20 ml methanol) and hydrogen peroxide (50 µl in 100 ml TBS). Dark purple precipitate bands indicate the presence of the antigens, specifically plasma proteins.

Duplicate samples of stroma-free hemoglobin, invention Hb, IEF-invention Hb, purified Hb from the Walter Reed Army Institute (WRAIR) (purified as described in the aforementioned Winslow et.al. paper) and purified Hb from Sigma Corporation (as described in their current catalogue of diagnostic reagents as "Hemoglobin Ao, reference H 0267) were focussed on the IEF gel. Half of the gel was stained with Coomassie-Blue, and the other half was blotted onto nitro cellulose paper. The blot was then stained to visualize human plasma-human plasma antibody complexes.

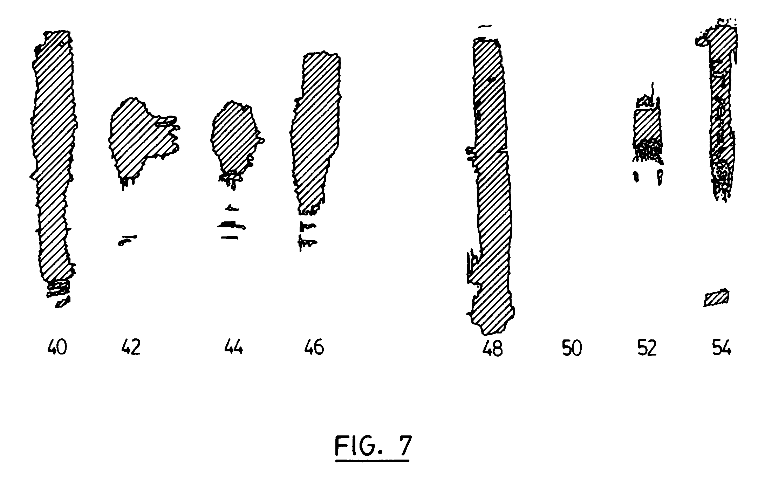

The results are shown in Fig. 7. In this Figure, lane 40 is the IEF Coomassie Blue stain of stroma free hemoglobin and lanes 42, 44 and 46 are the corresponding lanes from HbAo according to the invention, WRAIR Hb and Sigma Hb respectively. Lane 48 is the Western Blot - human plasma antibody test of stroma free hemoglobin, and lanes 50, 52 and 54 are the corresponding lanes from HbAo according to the invention, WRAIR Hb and Sigma Hb respectively.

Significant amounts of plasma proteins were detected in SFH. In contrast, only very minute traces of human serum albumin (HSA) were detected in the invention product lane. No other plasma proteins were detected in the Invention product. No detectable levels of HSA were found in the WRAIR HbAo, but numerous other plasma proteins above and below the HbAo bands were detected. Sigma HbAo standard contains numerous plasma contaminants including significant concentrations of HSA.

### EXAMPLE 14 - ACUTE HYPOVOLEMIC STUDY IN CONSCIOUS RATS

The product of the present invention was assayed for vasopressor activity.

Sprague-Dawley rats, randomly divided in groups of 6 (n=6), were allowed to acclimatize for four days, at which time, chronic cannulation was performed using the technique of Tabata and Chang⁽⁶⁾. Properly cannulated animals received intravenous heparin, 150 IU/kg. One arterial cannula was connected to a Stratham pressure transducer for blood pressure and heart rate. These parameters were recorded continuously on a Grass polygraph recorder. After steady baseline recording, lethal hemorrhagic shock was induced according to the method of Wiggers which involved the removal of 67% total blood volume through the other arterial catheter at 0.5 ml/min. in 2 stages. Immediately after shock induction, each rat received a test material at a rate of 0.5 ml/min, i.e. homologous blood, HbAo according to the invention (HbAo), or crosslinked HbAo according to the invention which had been reacted with o-raffinose crosslinking reagent as described in aforementioned U.S. patent application 08/231,945 filed April 21, 1994, (volume x3). Mean arterial pressure (MAP) and heart rate were recorded up to 30 min. after completion of infusion. Each rat was returned to a separate cage and monitored for 14 days for body weight, hematocrit and survival.

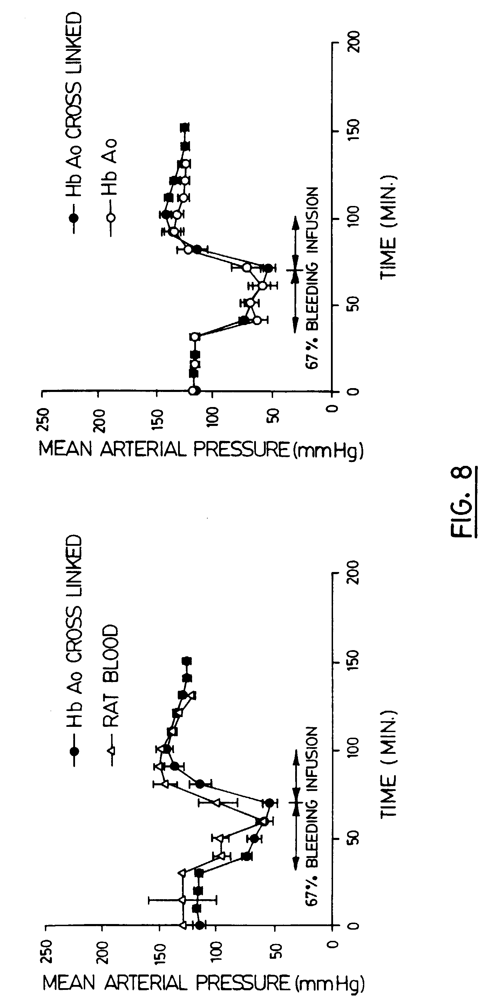

The results are shown graphically, on Figure 8, where MAP is plotted against time.

The data show that the product of the invention is a highly purified form of hemoglobin Ao that is as effective as whole blood in restoring mean arterial pressure after hemorrhagic shock in a conscious rat model. No differences in restored arterial pressure or heart rate were observed between the whole blood and HbAo treated groups.

### EXAMPLE 15 - HEMODYNAMIC EFFECTS OF ISOVOLEMIC EXCHANGE TRANSFUSIONS IN RATS

The HbAo of the present invention was assayed to evaluate hemodynamic effects upon isovolemic exchange transfusion in conscious rats.

The external iliac vein and artery of rats were cannulated via the femoral region, see Keipert and Chang.⁽⁸⁾ After a 24 hour recovery period, conscious animals were subjected to continuous 50% isovolemic exchange transfusions. During the exchange transfusion, the volume of blood removed through the iliac artery was replaced by test solution through the iliac vein at a rate of 0.5 ml/min using a constant flow infusion pump. Blood pressure and heart rate were monitored continuously: (1) for 30 min. prior to exchange, (2) during, and (3) for 2 - 3 hours after exchange. Mean arterial pressure and heart rate were calculated from the blood pressure trace.

One group of rats (four in number) were transfused with human serum albumin, HSA. For another group of rats (six in number), stroma-free hemoglobin (SFH) was the test solution. For another group of rats (eight in number) the product of the invention HbAo was the test solution.

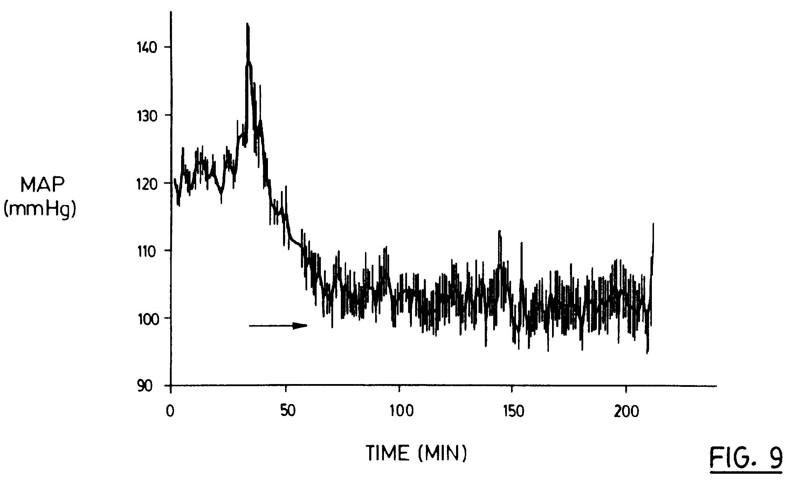

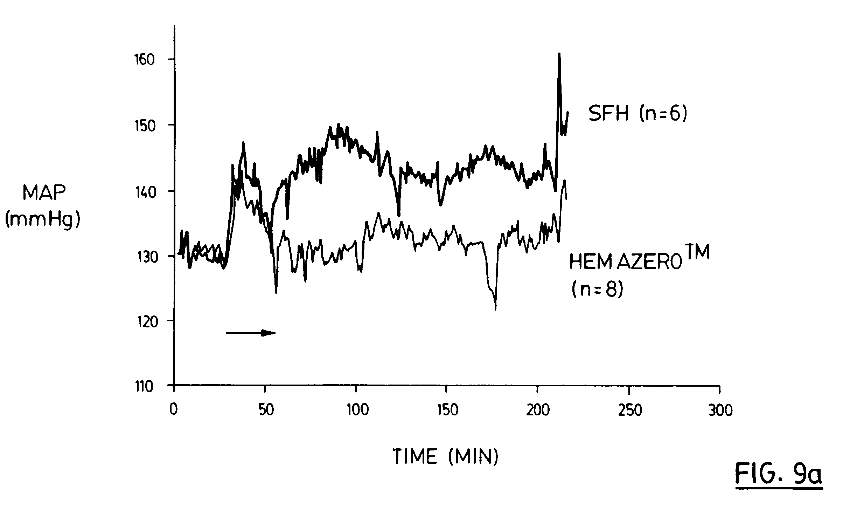

The results are presented graphically, on Figs. 9 and 9a.

For all test solutions, mean arterial pressure increased 10-20 mm Hg during the exchange transfusion procedure. With an albumin control solution, mean arterial pressure decreased 15 mm Hg below baseline during the rest of the post exchange monitoring period and remained at the decreased level for the entire post-exchange monitoring period of approximately 2.5 hours. (Figure 9). These observations are consistent with previously published observations by Keipert and Chang.⁽⁸⁾

With stroma-free hemoglobin, mean arterial pressure decreased back to baseline at the end of the exchange period but increased during the post-exchange monitoring period to a maximum of ∼15 mm Hg above baseline at 30-40 minutes post-exchange (Figure 9a).

In contrast, with HbAo of the present invention, the mean arterial pressure following exchange returned to baseline and increased post exchange by no more than 5 mm Hg thereby maintaining MAP within normal ranges.

The results of this study indicate that the HbAo in the invention is devoid of the potent vasoactivity associated with exchange transfusion with stroma-free hemoglobin.

### EXAMPLE 16 - AN IN VITRO TISSUE CULTURE ASSAY TO MEASURE OXIDATIVE INJURY IN ENDOTHELIAL CELLS PREVIOUSLY EXPOSED TO HEMOGLOBIN PREPARATIONS

The HbAo of the present invention was assayed for properties that may induce oxidative injury to endothelial cells in culture. The release of lactate dehydrogenase activity in the medium is a marker for cellular injury and cytotoxicity.

Endothelial cells from porcine thoracic aorta were cultured to confluence, subcultured in a 1:3 ratio in repeated passages. Within 48 hours of passage 7, cells were used for the experiment. Cellular injury was assessed by measuring the amount of lactate dehydrogenase released from the cells into the medium. Before hemoglobin solutions were added, the medium was removed and the cells were washed twice with phosphate buffered saline. Cells were incubated with hemoglobins (250 µM heme) in complete EGM medium for 6 or 24 hours at 37°C (n=4-6). Cells incubated in complete EGM medium alone served as the negative control. At the end of the incubation period, 50 µl of medium from each well were removed and lactate dehydrogenase activity was determined by a lactate dehydrogenase assay (Sigma). Lactate dehydrogenase activity in each well was compared with that released from cells after addition of 2% Triton X-100 (total LDH release) and expressed as percent total release.

After 6 hours incubation, a significant increase in lactate dehydrogenase release was observed in the presence of stroma-free hemoglobin SFH (74.4 ± 4.2%, p<0.005). A similar but smaller increase was observed with purified hemoglobin from the Walter Reed Army Institute of Research. No increase was observed with purified HbAo of the invention (39.8 ± 2.3%) when compared with the control (37.5 +/-2.2%). Lactate dehydrogenase release was further increased after 24 hours of incubation in the presence of stroma-free hemoglobin (99.6 ± 3.5%) whereas no change was observed with HbAo of the invention (41.6 ± 4.6%) compared to the control (34.1 ± 2.6%).

The incubation of cultured endothelial cells with HbAo, as prepared by the methods described in this invention, did not mediate the release of lactate dehydrogenase activity, a marker for cellular injury. Consequently, cytotoxicity to cultured endothelial cells is not an intrinsic property of hemoglobin solutions and the purification of HbAo removes cytotoxic components present in stroma-free hemoglobin.

### EXAMPLE 17 - REMOVAL OF PROCOAGULANT ACTIVITIES

The product of the present invention was assayed for its content of agents that potentiate the coagulation of blood. The method used is that of Biessels et al.⁽⁹⁾

Guinea pigs were anesthetized with Hyponorm™ and a silastic cannula inserted in the arteria carotis. After cannulation 30% of the blood volume was withdrawn (calculated as 2% of the body weight) and replaced with an equal volume of test solution. A 1% human albumin solution was infused at 2.5 ml per hour to maintain access to the cannula. The start of the albumin infusion was taken as the start of the experiment. Fibrino peptide A (FPA), known to be produced from fibrinogen upon the coagulation of blood, was measured before and following the infusion of Factor Xa (8-9 micrograms/kg), a dose that does not, in itself, cause coagulation. Blood samples were collected into an anticoagulant mixture of heparin (1000 U/ml), Trasylol (1000 U/ml) and sodium citrate (3.8%), separated and the plasma stored on ice.

Plasma samples were processed as described for the assay of FPA according to the method of Leessma, 1984⁽¹⁰⁾ and the formed FPA was measured using a RIA method previously published by Gerrits⁽¹¹⁾. Plasma proteins were precipitated with an equal volume of 40% PEG 6000 in phosphate buffered saline. Supernatant solutions were heated in a boiling water bath, centrifuged and stored for assay. The results are shown below, in Table 8.

**TABLE 8**

| Test Sample | Description | AUC₁₀* |
|---|---|---|
| SFH | Rapid, transient FPA increase from 1.4 ng/ml to 13.8 +/-3 ng/ml within the first 10 minutes followed by a decline to baseline after 40 minutes. | 87.6 +/-12 |
| HSA | Increase in FPA from baseline of 1.4 ng/ml to a maximum of 5.5 +/-1.5 ng/ml. | 45.7 +/-8.2 |
| HbAo | Increase in FPA from baseline of 1.4 ng/ml to a maximum of 5.0 +/- 1.2 ng/ml | 31.8 +/-2.9 |

| | | |
|---|---|---|
| * Area under the curve in the initial 10 minutes after administration of the Factor Xa. | | |

These data show that the product of the present invention has essentially no procoagulant activity, as compared with stroma-free hemoglobin. Such procoagulant activity is believed to be based largely on contamination of the test solutions with lipids and phospholipids.

### EXAMPLE 18 - QUANTITATION OF CONTAMINATING HUMAN SERUM PROTEINS BY ELISA TECHNIOUES

The amount of serum protein contaminants in the purified hemoglobin product of the present invention was measured and compared with that of other available hemoglobin products, by a sandwich ELISA technique.

In the present Example, a sandwich ELISA technique according to the general procedures and techniques of Butler, J.E. (12) was used.

Dynatech 96 well, 1 mm 4 microtiter plates were coated with solid phase goat serum absorbed Rabbit anti-Human Serum antibody (Dako Corporation, Santa Barbara, CA, Lot #072), diluted 1:40 in Tris-buffered saline. After incubation for 2 hours at 37°C, the plate was washed with Tris-buffered saline (TBS) and the remaining free sites on the plate were blocked by incubating with hydrolyzed fish gelatin (Hipure Liquid Gelatin, Norland Products Inc., New Brunswick, N.J.). After incubation for one hour, the plate was washed with TBS, and test articles were added using two-fold serial dilutions. After incubation for one hour the plate was washed again with TBS and incubated with solid rabbit phase serum coated Goat anti-Human Serum antibody, IgG fraction (Accurate Chemical and Scientific Corporation, Westbury N.Y., Lot #H036) containing 1% fish gelatin, diluted 1:1000 in TBS. After incubation for 1 hour, the plate was washed with TBS and then incubated with affinity purified Rabbit anti-Goat antibody, IgG fraction (Horseradish Peroxidase HRP conjugated) (BioRad Lot #75312) diluted 1:250 in TBS. After incubation for two hours the plate was washed with TBS. The antigen-antibody-HRP complex was visualized by using o-phenyldiamine-dichloride (OPD - 26 mg/15 ml citric acid, pH 5.0). Pale yellow colours indicate the presence of antigens, specifically serum proteins. Their absorbance was read using a Molecular Devices Thermomax Microplate reader at 490 nm.

Duplicate samples of normal human serum (Dimension Laboratories Inc., Mississauga, Ontario, Lot # 30317), purified HbAo from Sigma Corporation (H-0267), purified HbAo from the Walter Reed Army Institute of Research (WRAIR) (Lot #92092) and purified HbAo of the invention were assayed. The results are summarized in Table 9. It is apparent that the product of the invention has no more than 0.0004% serum proteins, whereas the other tested samples are at least an order of magnitude higher in this regard.

**TABLE 9**

| **SAMPLE** | **SERUM PROTEIN CONTENT (wt% based on Hb)** | **SERUM PROTEIN AS MILLIGRAMS PER ML OF SOLUTION** |
|---|---|---|
| HbAo of this invention (Solution of 8% total Hb) | 0.0004% | 0.000328 mg/ml |
| WRAIR HbAo (Solution of 6.7% total Hb) | 0.0039% | 0.00263 mg/ml |
| SIGMA HbAo (Solution of 1.1% total Hb) | 0.0029% | 0.000328 mg/ml |

In all of the assays conducted, the only serum protein detected in the product according to the invention was human serum albumin. The serum proteins found in the other, competitive products appear to be heterogeneous mixtures comprising HSA and other serum proteins.

### REFERENCES

1. Hess et al. "Systemic & Pulmonary Hypertension After Resuscitation with Cell-Free Hemoglobin" 1993.
2. Change et al., "Effect of Single Replacement ...", BIOMAT, Art Cells and Immob. Biotech, 20(2-4), pp 503-510, (1992).
3. Simoni et al., "Reaction of Human Endothelial Cells to Bovine Hemoglobin Solutions and Tumor Necrosis Factor", Art. Cells, Blood Subs., and Immob. Biotech." 22(3):777-787, 1992.
4. Huisman et al., "Studies on the Heterogeneity of Hemoglobins. IX - The Use of Tris-Hcl Buffers in the Anion-Exchange Chromatography of Hemoglobins", J. Chromatog., 19, 160-169, (1965).
5. Savavis, C.A. and Zamcheck, N. "Isoelectric Focusing in Agarose", Journal of Immunological Methods 29:91-96, 1979.
6. Tabata and Change, Artificial Organs, 6 213-214 (1982).
7. Wigger, C.J., "Physiology of Shock", New York, The Common Fund, pp 138-139 (1950).
8. Keipert and Chang, "Effects of Partial ...", Vox. Sang 53, 7-14 (1987).
9. Biessels et al., Annual Report of the Karl Landsteiner Foundation, 1991.
10. Leeksma et al., Blood 67: 650-654, 1986.
11. Gerrits et al., Thromb. Res 5: 197, 1974.
12. Butler, J. E., ELISA and other Solid Phase Immunoassays, edited by D.M.Kemeny and S.J.Challacombe, John Wiley and Sons Ltd, 1988, pp1-180.

## Claims

1. A process of separating a preselected hemoglobin from a crude solution thereof which also contains contaminating proteinaceous substances, said process comprising:
in a first chromatographic stage, feeding the crude solution to a chromatographic column and subjecting it to chromatography under either anion exchange conditions under which components of the crude solution more acidic than said preselected hemoglobin have preferential binding affinity thereover, or to cation exchange conditions under which components of the crude solution more basic than said preselected hemoglobin have preferential binding affinity thereover;
continuing the feed of the crude solution in the first chromatographic stage to and beyond the stage where the column is fully loaded with hemoglobin species and components of greater affinity, to cause column overload and subsequent displacement of the hemoglobin species therefrom;
in a second chromatographic stage, feeding the hemoglobin species-containing eluent from he first stage to a chromatographic column and subjecting it to chromatography under said anion exchange conditions or cation exchange conditions not selected for said first stage;
continuing the feed of said eluent in the second chromatographic stage to and beyond the stage where the column is fully loaded with hemoglobin species and components of greater affinity under said second stage conditions to cause column overload and consequent displacement of the hemoglobin species therefrom.

2. The process of claim 1 wherein the first stage is anion exchange chromatography, and the second stage is cation exchange chromatography.

3. The process of claim 2 wherein the preselected hemoglobin species is normal adult human hemoglobin HbAo.

4. The process of claim 3 wherein the first stage, anion exchange process is conducted at pH 7 - 10 and using a feed solution of low conductivity, less than 3 mS.

5. The process of claim 4 wherein the first stage, anion exchange process is conducted at pH 8.5 - 9.0 and using a feed solution of conductivity less than 1 mS.

6. The process of claim 4 wherein the feed solution is fed to the column at a rate not greater than 10 cm per minute.

7. The process of claim 6 wherein the feed solution concentration is in the range 0.1 - 20%.

8. The process of claim 4 wherein the second stage, cation exchange chromatography is conducted at pH 6.5 - 8.5 using a feed solution of conductivity less than 3 mS.

9. The process of claim 8 wherein the second state, cation exchange chromatography is conducted at pH 7 - 7.5 using a feed solution of conductivity less than 1 mS.

10. The process of claim 8 wherein the feed solution is fed to the second stage, cation exchange column at a rate not greater than 10 cm per minute.

11. The process of claim 8 wherein concentration of the feed solution to the second stage cationic exchange column is from 2 - 6%.

12. An aqueous solution of adult human hemoglobin HbAo, suitable for use as a blood substitute, said solution comprising as solute at least 99.5% HbAo, and being free from detectable, active lipid encapsulated viruses, as determined by standard tests.

13. A highly pure hemoglobin product comprising an aqueous solution of human hemoglobin free of all red blood cell membrane proteins as determined by isoelectric focusing and immunoblot analysis, a human serum albumin content of less than 5µg per 100 mg of hemoglobin.

14. Purified adult hemoglobin HbAo having a content of human serum protein which is not greater than 0.0005 wt. %, based on the total weight of HbAo, and being free of detectable amounts of other proteinaceous materials.

15. Purified adult hemoglobin HbAo as claimed in claim 15 which is additionally free from detectable, active lipid encapsulated viruses.

## Patentansprüche

1. Verfahren zur Aufreinigung eines bestimmten Hemoglobins aus einer Roh-Lösung davon, die zusätzlich Protein-Verunreinigungen enthält, welches umfaßt:
die Rohlösung in einem ersten chromatographischen Schritt auf eine Chromatographiesäule aufzutragen und diese einer Chromatographie zu unterziehen unter entweder Anionenaustausch-Bedingungen, bei denen die Komponenten der Rohlösung, die saurer sind als das bestimmte Hemoglobin, eine bevorzugte Affinitätsbindung aufweisen, oder bei Kationenaustausch-Bedingungen, bei denen die Komponenten der Roh-Lösung, die basischer sind als das bestimmte Hemoglobin, eine bevorzugte Bindungsaffinität aufweisen,
die Rohlösung in dem ersten chromatographischen Schritt bis zu und über einen Zustand weiter aufzutragen, bei dem die Säule mit der Hemoglobin-Spezies und Komponenten mit höherer Affinität vollständig beladen ist, wobei eine Überbeladung der Säule bewirkt wird und die Hemoglobin-Spezies anschließend davon entfernt wird,
den die Hemoglobin-Spezies enthaltenden Ausfluß aus dem ersten Schritt in einem zweiten Schritt auf eine Chromatographiesäule aufzutragen und diese einer Chromatographie unter den für den ersten Schritt nicht gewählten Anionenaustausch-Bedingungen oder Kationenaustausch-Bedingungen zu unterziehen,
den Ausfluß in dem zweiten chromatographischen Schritt bis zu und über einen Zustand weiter aufzutragen, bei dem die Säule mit der Hemoglobin-Spezies und Komponenten höherer Affinität unter den Bedingungen des zweiten Schritts vollständig beladen ist, um eine Überbeladung der Säule zu bewirken und infolgedessen eine Entfernung der Hemoglobin-Spezies davon zu bewirken.

2. Verfahren nach Anspruch 1, wobei der erste Schritt eine Anionenaustauschchromatographie und der zweite Schritt eine Kationenaustauschchromatographie ist.

3. Verfahren nach Anspruch 2, wobei die bestimmte Hemoglobin-Spezies normales menschliches Erwachsenen-Hemoglobin HbA0 ist.

4. Verfahren nach Anspruch 3, wobei der erste Schritt, das Anionenaustauschverfahren, bei einem pH-Wert von 7 - 10 und unter Verwendung einer Beschickungslösung mit geringer Leitfähigkeit von weniger als 3 mS durchgeführt wird.

5. Verfahren nach Anspruch 4, wobei der erste Schritt, das Anionenaustauschverfahren bei einem pH-Wert von 8,5-9,0 und unter Verwendung einer Beschickungslösung mit einer Leitfähigeit von unter 1 mS durchgeführt wird.

6. Verfahren nach Anspruch 4, wobei die Beschickungslösung auf die Säule in einer Geschwindigkeit von nicht über 10 cm / min aufgetragen wird.

7. Verfahren nach Anspruch 6, wobei die Konzentration der Beschickungs-Lösung im Bereich von 0,1 - 20 % liegt.

8. Verfahren nach Anspruch 4, wobei der zweite Schritt, die Kationenaustauschchromatographie bei einem pH-Wert von 6,5 - 8,5 unter Verwendung einer Beschickungslösung mit einer Leitfähigkeit von unter 3 mS durchgeführt wird.

9. Verfahren nach Anspruch 8, wobei der zweite Schritt, die Kationenaustauschchromatographie bei einem pH-Wert von 7 - 7,5 unter Verwendung einer Beschickungslösung mit einer Leitfähigkeit von unter 1 mS durchgeführt wird.

10. Verfahren nach Anspruch 8, wobei die Beschickungslösung dem zweiten Schritt, der Kationenaustauschsäule, in einer Geschwindigkeit von nicht über 10 cm / min zugeführt wird.

11. Verfahren nach Anspruch 8, wobei die Konzentration der Beschickungslösung zu dem zweiten Schritt, der Kationenaustauschsäule, im Bereich von 2 - 6 % liegt.

12. Wäßrige Lösung von menschlichem Erwachsenen-Hemoglobin HbA0, das zur Verwendung als Blutersatzstoff geeignet ist, wobei die Lösung als Gelöstes mindestens 99,5 % HbA0 enthält und frei ist von nachweisbaren, aktiven, mit Lipid verkapselten Viren, bestimmt gemäß Standarduntersuchungen.

13. Äußerst reines Hemoglobin-Produkt, welches eine wäßrige Lösung von menschlichem Hemoglobin enthält, das frei ist von allen Membranproteinen roter Blutkörperchen, bestimmt anhand isolelektrischer Fokussierung und Immunoblot-Analyse, und einen Gehalt an menschlichem Serumalbumin von weniger als 5 µg / 100 mg Hemoglobin aufweist.

14. Gereinigtes Erwachsenen-Hemoglobin HbA0 mit einem Gehalt an menschlichem Serumprotein, der 0,0005 Gew.-% nicht übersteigt, bezogen auf das Gesamtgewicht an HbA0, und frei ist von nachweisbaren Mengen anderer Proteinmaterialien.

15. Gereinigtes Erwachsenen-Hemoglobin HbA0 nach Anspruch 15, das zusätzlich frei ist von nachweisbaren, aktiven, mit Lipid verkapselten Viren.

## Revendications

1. Procédé pour séparer une hémoglobine présélectionnée d'une solution brute de celle-ci qui contient aussi des substances protéiniques contaminantes, ledit procédé consistant :
dans une première étape chromatographique, à introduire la solution brute dans une colonne chromatographique et à la soumettre à une chromatographie soit dans des conditions d'échange d'anions dans lesquelles les composants de la solution brute davantage acides que ladite hémoglobine présélectionnée ont une affinité de fixation préférentielle par rapport à celle-ci, soit dans des conditions d'échange de cations dans lesquelles les composants de la solution brute davantage basiques que ladite hémoglobine présélectionnée ont une affinité de fixation préférentielle par rapport à celle-ci ;
à poursuivre l'introduction de la solution brute dans la première étape chromatographique jusqu'au stade où la colonne est complètement chargée d'espèce d'hémoglobine et de composants de plus forte affinité, et au-delà de ce stade, de façon à provoquer une surcharge de la colonne et un déplacement subséquent de l'espèce d'hémoglobine depuis celle-ci ;
dans une deuxième étape chromatographique, à introduire l'éluant contenant l'espèce d'hémoglobine provenant de la première étape dans une colonne chromatographique et à le soumettre à une chromatographie dans les conditions d'échange d'anions ou les conditions d'échange de cations non sélectionnées pour la première étape ;
à poursuivre l'introduction de l'éluant dans la deuxième étape chromatographique jusqu'au stade où la colonne est complètement chargée d'espèce d'hémoglobine et de composants de plus forte affinité dans les conditions de la deuxième étape, et au-delà de ce stade, de façon à provoquer une surcharge de la colonne et un déplacement subséquent de l'espèce d'hémoglobine depuis celle-ci.

2. Procédé selon la revendication 1, dans lequel la première étape est une chromatographie par échange d'anions, et la deuxième étape est une chromatographie par échange de cations.

3. Procédé selon la revendication 2, dans lequel l'espèce d'hémoglobine présélectionnée est l'hémoglobine normale de l'être humain adulte HbAo.

4. Procédé selon la revendication 3, dans lequel le procédé d'échange d'anions de première étape est réalisé à un pH de 7 à 10 et utilise une solution de charge de faible conductivité, inférieure à 3 mS.

5. Procédé selon la revendication 4, dans lequel le procédé d'échange d'anions de première étape est réalisé à un pH de 8,5 à 9,0 et utilise une solution de charge de conductivité inférieure à 1 mS.

6. Procédé selon la revendication 4, dans lequel la solution de charge est introduite dans la colonne à un débit ne dépassant pas 10 cm par minute.

7. Procédé selon la revendication 6, dans lequel la concentration de la solution de charge est située dans la plage allant de 0,1 à 20 %.

8. Procédé selon la revendication 4, dans lequel la chromatographie par échange de cations de deuxième étape est réalisée à un pH de 6,5 à 8,5 et utilise une solution de charge ayant une conductivité inférieure à 3 mS.

9. Procédé selon la revendication 8, dans lequel la chromatographie par échange de cations de deuxième étape est réalisée à un pH de 7 à 7,5 et utilise une solution de charge ayant une conductivité inférieure à 1 mS.

10. Procédé selon la revendication 8, dans lequel la solution de charge est introduite dans la colonne d'échange de cations à un débit ne dépassant pas 10 cm par minute.

11. Procédé selon la revendication 8, dans lequel la concentration de la solution de charge de la colonne d'échange de cations de deuxième étape est située dans la plage allant de 2 à 6 %.

12. Solution aqueuse d'hémoglobine de l'être humain adulte HbAo, utilisable en tant que substitut du sang, la solution comprenant, en tant que soluté, au moins 99,5 % de HbAo, et étant exempte de virus encapsulés dans des lipides actifs détectables, comme déterminé par des essais standard.

13. Produit de type hémoglobine très pure, comprenant une solution aqueuse d'hémoglobine humaine exempte de toutes protéines de membrane de globule rouge, comme déterminé par focalisation isoélectrique et analyse par immunotransfert, et ayant une teneur en sérumalbumine humaine inférieure à 5 µg pour 100 mg d'hémoglobine.

14. Hémoglobine adulte purifiée HbAo ayant une teneur en protéine sérique humaine qui n'est pas supérieure à 0,0005 % en poids par rapport au poids total de HbAo, et qui est exempte de quantités détectables d'autres matériaux protéiniques.

15. Hémoglobine adulte purifiée HbAo selon la revendication 15, qui est de plus exempte de virus encapsulés dans des lipides actifs détectables.
